Europäisches Patentamt

European Patent Office

Office européen des brevets

(11) Numéro de publication: **0 245 153**
**A1**

## DEMANDE DE BREVET EUROPEEN

(21) Numéro de dépôt: 87400957.4

(22) Date de dépôt: 24.04.87

(51) Int. Cl.⁴: **H 05 G 1/08,** H 05 G 1/70, H 04 B 9/00

(30) Priorité: 06.05.86 FR 8606545

(43) Date de publication de la demande: 11.11.87 Bulletin 87/46

(84) Etats contractants désignés: DE GB IT NL

(71) Demandeur: THOMSON-CGR, 13, square Max-Hymans, F-75015 Paris (FR)

(72) Inventeur: Audon, Philippe, THOMSON-CSF SCPI 19, avenue de Messine, F-75008 Paris (FR)
Inventeur: Courtecuisse, Daniel, THOMSON-CSF SCPI 19, avenue de Messine, F-75008 Paris (FR)

(74) Mandataire: Grynwald, Albert et al, THOMSON-CSF SCPI 19, avenue de Messine, F-75008 Paris (FR)

(54) **Installation de radiologie à plusieurs appareils commandés selon une boucle avec un dispositif de courtcircuit pour chaque appareil.**

(57) Installation de radiologie, notamment radioscopie et radiographie, comportant plusieurs appareils entre lesquels les signaux de commande et d'état sont transmis en série selon une boucle. Un dispositif de commutation (22) est associé à chaque appareil afin que la boucle reste fermée quand cet appareil ne fonctionne pas.

Le dispositif de commutation comporte, à l'entrée de l'appareil correspondant, un simple interrupteur (23) qui est ouvert quand l'appareil fonctionne et fermé dans le cas contraire. Les informations sont transmises sous forme de signaux électriques par l'appareil dans le premier cas et par l'intermédiaire de l'interrupteur fermé (23) dans le second cas.

# INSTALLATION DE RADIOLOGIE A PLUSIEURS APPAREILS COMMANDES SELON UNE BOUCLE AVEC UN DISPOSITIF DE COURT-CIRCUIT POUR CHAQUE APPAREIL.

L'invention est relative à une installation de radiologie, notamment de radiographie ou radioscopie, comportant plusieurs appareils entre lesquels des signaux de commande ou d'état sont transmis en série selon une boucle.

Une installation de radiographie ou de radiologie est souvent commandée à partir d'un pupitre auquel parviennent les informations (signaux d'état) provenant des divers appareils tels que : générateur de rayons X, amplificateur de luminance, caméra de télévision, appareil photographique, etc... et duquel partent les signaux de commande de ces appareils.

La configuration la plus simple pour le transfert d'informations est la disposition en série : les signaux d'état et de commande transitent d'un appareil à un autre. Quand un appareil n'est pas utilisé ou est en panne, un dispositif de commutation, qui est associé à chaque appareil, permet de court-circuiter ce dernier afin que la transmission des signaux ne soit pas perturbée par l'arrêt ou la panne de cet appareil. Une telle installation est décrite dans le brevet européen N° 136 645. Les dispositifs de commutation utilisés dans l'installation de cette publication sont du type à fibre optique. Ils sont complexes, onéreux et relativement fragiles.

L'invention remédie à ces inconvénients.

Elle est caractérisée en ce que le dispositif de commutation comporte, à l'entrée de l'appareil correspondant, un simple interrupteur pour signaux électriques qui est ouvert quand l'appareil fonctionne et est fermé, pour assurer la continuité de la ligne, en cas de non-fonctionnement de cet appareil.

Dans un exemple, la ligne de transmission est un conducteur électrique et l'interrupteur peut ainsi être disposé entre l'entrée et la sortie de l'appareil soit à l'intérieur, soit à l'extérieur de ce dernier. De préférence, pour simplifier la construction, l'interrupteur est à l'intérieur de l'appareil dans le même boitier, avantageusement sur le même circuit imprimé que le reste des éléments de ce dernier.

Dans une autre réalisation, les informations sont transmises d'un appareil à un autre par une fibre optique et, ainsi, à l'entrée de l'appareil, on prévoit un convertisseur de lumière en signal électrique et à sa sortie, un convertisseur complémentaire signal électrique/signal lumineux. Dans ce cas, les interrupteurs des dispositifs de commutation se trouvent entre la sortie du convertisseur d'entrée et l'entrée du convertisseur de sortie.

D'autres caractéristiques et avantages de l'invention apparaitront avec la description de certains de ses modes de réalisation, celle-ci étant effectuée en se référant aux dessins annexés sur lesquels :

- la figure 1 est un schéma d'une installation selon l'invention, et

- la figure 2, est un schéma d'un dispositif de commutation de l'installation de la figure 1.

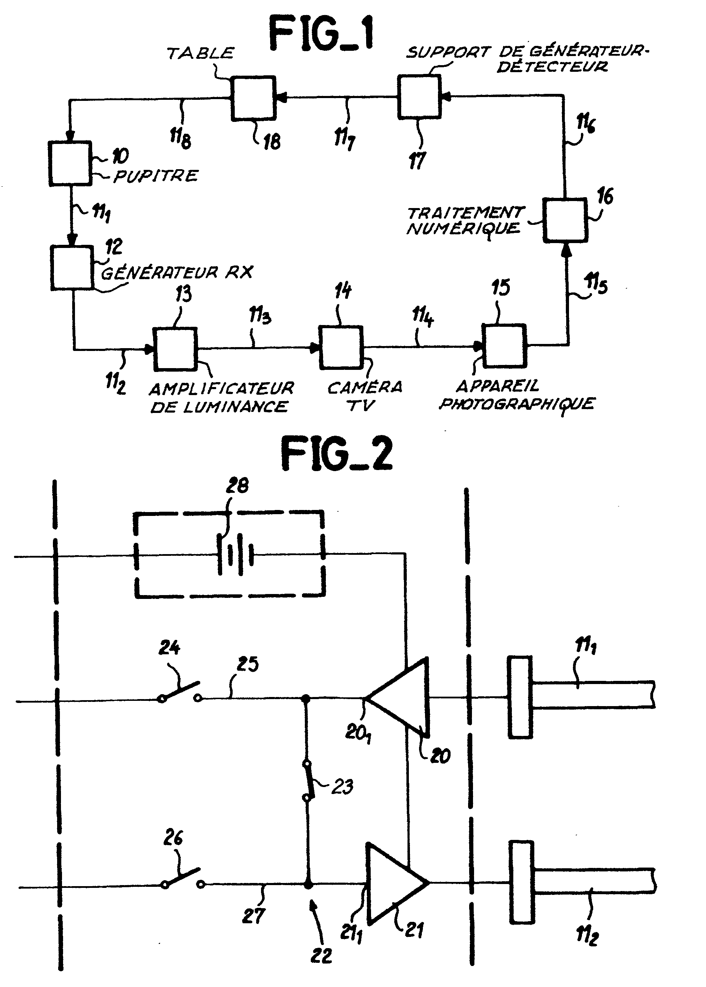

Dans l'exemple représenté sur la figure 1 les informations de commande de l'installation de radiographie ou radioscopie sont émises à partir d'un pupitre 10 qui, comme tous les autres appareils de cette installation, présente, à son entrée, un convertisseur 20 (figure 2) de signal lumineux en signal électrique et, à sa sortie, un convertisseur 21 de signal électrique en signal lumineux. Les signaux de commande qu'émet ce pupitre sont transmis par une fibre optique $11_1$ vers un second appareil 12 qui est ici un générateur de rayons X. Les informations fournies par le conducteur de lumière $11_1$ repartent de l'appareil 12 pour être acheminées, par une autre fibre optique $11_2$, vers un troisième appareil 13 tel qu'un amplificateur de luminance ; en outre cette fibre $11_2$ transmet des informations fournies par l'appareil 12 (par exemple des informations relatives à l'état de cet appareil qui seront acheminées vers le pupitre 10 pour l'information de l'opérateur).

L'appareil utilise, le cas échéant, les informations reçues et, de toute façon, les transmet à une autre fibre optique $11_3$. Ensuite, on prévoit une caméra de télévision 14, un appareil photographique 15, un dispositif 16 de traitement numérique de l'image, un arceau 17 portant le générateur de rayons X et un détecteur, et une table 18 porte-patient connectée au pupitre 10. La transmission d'informations est effectuée par d'autres fibres optiques respectivement : $11_4$, $11_5$, $11_6$, $11_7$ et $11_8$. Cette dernière fibre optique transmet les informations de l'appareil 18 au pupitre 10.

A chaque appareil (10, 12, 13, 14, 15, 16, 17, 18), en plus des convertisseurs 20 et 21, est associé un dispositif de commutation 22 qui comprend un simple interrupteur 23 disposé entre la sortie $20_1$ du convertisseur 20 et l'entreé $21_1$ du convertisseur 21. En outre, un interrupteur 24 est en série avec le conducteur 25 à la sortie $20_1$ du convertisseur 20 transmettant les informations à l'appareil correspondant, par exemple celui de référence 12 ; de même un interrupteur 26 est en série avec le conducteur 27 provenant de l'appareil 12 et pénétrant dans le convertisseur 21.

Une pile électrique 28 est prévue pour alimenter les convertisseurs 20 et 21 même en cas de panne de l'alimentation de l'appareil correspondant 12. Ainsi, de préférence, cette pile est distincte de l'alimentation des autres éléments de circuit de l'appareil.

En fonctionnement normal l'interrupteur 23 est ouvert et les interrupteurs 24 et 26 sont fermés. Ainsi les informations provenant de la fibre $11_1$ sont acheminées à l'appareil qui exécute, le cas échéant, la commande détectée et des informations sont retransmises vers le conducteur 27 et, de là, par la fibre $11_2$, vers les autres appareils.

En cas d'incident, l'interrupteur 23 est fermé. De cette manière les informations sont transmises directement de la sortie $20_1$ du convertisseur 20 vers l'entrée $21_1$ du convertisseur 21 sans perturber le fonctionnement des autres appareils et la transmission d'informations.

4

Les trois interrupteurs 23, 24 et 26 sont par exemple commandés par une bobine de relais (non représentée).

Dans l'exemple la pile 28, les convertisseurs 20 et 21, ainsi que les interrupteurs 23, 24 et 26 sont disposés sur une même plaquette de circuit imprimé à l'intérieur du boitier de l'appareil correspondant 12. Ces élements 20, 21, 23, 24, 26, 28 peuvent aussi être disposés sur la même plaquette de circuit que celle portant les autres éléments de circuit de l'appareil 12.

En variante, au lieu de fibres optiques $11_1$, $11_2$, on prévoit de simples conducteurs. Dans ce cas, il n'est, bien entendu, pas nécessaire de prévoir les convertisseurs 20 et 21, ni la pile 28.

L'ouverture des interrupteurs 24 et 26 et la fermeture de l'interrupteur 23 intervient par exemple, en cas d'interruption de l'alimentation en énérgie électrique de l'appareil 12 alors que les autres appareils restent alimentés.

Le dispositif de commutation 22 est d'une très grande simplicité, d'un faible coût et d'une grande fiabilité.

5

## REVENDICATIONS

1. Installation de radiologie comportant plusieurs appareils (10, 12, 13, 14... 18) entre lesquels les signaux de commande et d'état sont transmis en série selon une boucle, un dispositif de commutation étant associé à chaque appareil afin que la boucle reste fermée quand cet appareil ne fonctionne pas, caractérisée en ce que le dispositif de commutation (22) associé à chaque appareil comporte, à l'entrée de l'appareil correspondant, un simple interrupteur (23) qui est ouvert quand cet appareil fonctionne et fermé quand cet appareil ne fonctionne pas, les informations étant transmises sous forme de signaux électriques par l'appareil dans le premier cas et par l'intermédiaire de l'interrupteur fermé (23) dans le second cas.

2. Installation selon la revendication 1 caractérisée en ce que le dispositif de commutation comporte, en outre, un interrupteur (24) en série avec un conducteur (25) d'entrée et un interrupteur (26) en série avec un conducteur (27) de sortie.

3. Installation selon la revendication 2 caractérisée en ce que l'interrupteur (23) entre l'entrée et la sortie et les interrupteurs (24 et 26) en série avec les conducteurs d'entrée (25) et de sortie (27) sont commandés par une même bobine de relais.

4. Installation selon l'une quelconque des revendications précédentes caractérisée en ce que les informations sont transmises par des fibres optiques (11) entre les appareils et en ce que chaque dispositif de commutation comporte, à l'entrée, un convertisseur (20) de signal optique en signal électrique et, à la sortie, un convertisseur (21) de signal électrique en signal optique, l'interrupteur de courtcircuit (23) du dispositif de commutation reliant la sortie $(20_1)$ du convertisseur d'entrée (20) à l'entrée $(21_1)$ du convertisseur (21) de sortie.

5. Installation selon la revendication 4, caractérisée en ce qu'une pile électrique (28) alimente les convertisseurs (20, 21).

6. Installation selon la revendication 5, caractérisée en ce que la pile électrique (28) est distincte de l'alimentation des autres éléments du circuit de l'appareil correspondant.

7. Installation selon l'une quelconque des revendications précédentes caractérisée en ce que le dispositif de commutation est disposé à l'intérieur du boitier de l'appareil correspondant.

8. Installation selon la revendication 7, caractérisée en ce que le dispositif de commutation est disposé sur la même plaquette de circuit imprimé que d'autres éléments du circuit de l'appareil correspondant.

# FIG_1

TABLE

SUPPORT DE GÉNÉRATEUR-DÉTECTEUR

$11_8$

18

$11_7$

17

$11_6$

10 PUPITRE

$11_1$

12 GÉNÉRATEUR RX

TRAITEMENT NUMÉRIQUE

16

13

$11_3$

14

$11_4$

15

$11_5$

$11_2$

AMPLIFICATEUR DE LUMINANCE

CAMÉRA TV

APPAREIL PHOTOGRAPHIQUE

# FIG_2

28

24

25

$20_1$

20

$11_1$

23

26

27

$21_1$

21

$11_2$

22

0245153

**Office européen des brevets**

## RAPPORT DE RECHERCHE EUROPEENNE

Numéro de la demande

EP 87 40 0957

| | DOCUMENTS CONSIDERES COMME PERTINENTS | | |
|---|---|---|---|
| Catégorie | Citation du document avec indication, en cas de besoin, des parties pertinentes | Revendication concernée | CLASSEMENT DE LA DEMANDE (Int. Cl.4) |
| Y,D | EP-A-0 136 645 (SIEMENS AG) * Page 1, ligne 8 - page 2, ligne 23; page 3, ligne 20 - page 4, ligne 7; figures 1,2 * | 1,4 | H 05 G 1/08 H 05 G 1/70 H 04 B 9/00 |
| Y | DE-A-2 724 841 (PHILIPS PATENTVERWALTUNGS GmbH) * Page 1, lignes 1-18; page 7, ligne 16 - page 9, ligne 5; figure 2 * | 1 | |
| A | US-A-4 148 558 (D.B. SCHUCK) * Colonne 1, ligne 5 - colonne 2, ligne 8; figures 1,2 * & FR-A-2 406 351 | 1,4 | |
| A | DE-A-3 436 135 (FUJI ELECTRIC CO., LTD) * Page 2, ligne 1 - page 5, ligne 30; figures 1-4 * | 1,4 | DOMAINES TECHNIQUES RECHERCHES (Int. Cl.4) H 04 B H 05 G |
| A | FR-A-2 429 539 (SIEMENS AG) * Page 1, ligne 1 - page 2, ligne 6 * | 1,4 | |

Le présent rapport de recherche a été établi pour toutes les revendications

| Lieu de la recherche | Date d'achèvement de la recherche | Examinateur |
|---|---|---|
| LA HAYE | 14-08-1987 | HORAK G.I. |